# EUROPÄISCHE PATENTSCHRIFT

(11) **EP 2 385 797 B1**
(45) Veröffentlichungstag und Bekanntmachung des Hinweises auf die Patenterteilung: **11.11.2015**
(21) Anmeldenummer: 09812417.5
(22) Anmeldetag: 22.12.2009
(51) Int. Cl.: A61B 17/02, F16B 2/18, F16L 23/06

(54) **SCHNELLSPANNKLAMMER EINER WUNDSPREIZVORRICHTUNG**
QUICK-ACTION TENSIONING CLIP OF A WOUND RETRACTOR
PINCE DE SERRAGE RAPIDE D'UN ÉCARTEUR DE PLAIES

(30) Priorität: 22.12.2008 DE 102008064195
(43) Veröffentlichungstag der Anmeldung: 16.11.2011
(73) Patentinhaber: Condor GmbH Medicaltechnik, 33154 Salzkotten (DE)
(72) Erfinder: SCHULTE, Hermann-Josef, 33154 Salzkotten (DE)
(74) Vertreter: Rolf, Gudrun
(86) Internationale Anmeldenummer: PCT/DE2009/001800
(87) Internationale Veröffentlichungsnummer: WO 2010/072212

(56) Entgegenhaltungen:
- GB-A- 1 418 017
- GB-A- 1 472 750
- US-A1- 2007 213 597

## Beschreibung

Die Erfindung betrifft eine Schnellspannklammer einer Wundspreizvorrichtung mit mindestens einer verspannbaren Aufnahme für mindestens einen Haltearm oder eine Gelenkkugel eines Haltearmes gemäß dem Oberbegriff des Hauptanspruches.

Es ist eine Wundspreizvorrichtung bekannt, DE 19712746 C2, die einen Verbindungsarm zu einem Zentral halter eines OP-Tisches aufweist, mit einer daran angeordneten Schnellspannklammer die eine Welle hält, auf der zwei weitere Schnellspannklammern angeordnet sind, die ihrerseits Haltearme der Wundspreizvorrichtung tragen.

Die Spannvorrichtungen bestehen bei dieser bekannten Ausführung jeweils aus Schraubverschlüssen, die zu ungünstig ausgerichteten Betätigungshebeln führen können und zur sicheren Fixierung der Haltearme und deswegen auch zum Lösen einen hohen Kraftaufwand fordern. Andere bekannte Exzenter-Spannvorrichtungen weisen zum Teil schlecht dosierbare und nicht einstellbare Klemmeigenschaften auf.

Weitere bekannte Schnellspannklammern, US 2007/213507 A1, die eine Schnellspannklammer gemäß der Präambel von Anspruch 1 offenbart, und GB 1 418 017, besitzen jeweils nur zwei Positionen, eine Klemmposition und eine offene Position, wobei in der offenen Position die in der Klemmposition zu fixierenden Haltearme oder Gelenkkugeln unkontrolliert verschwenken können oder die Verbindung sogar komplett auseinanderfallen kann.

Aufgabe der Erfindung ist es, eine Schnellspannklammer zur Verfügung zu stellen, die sich sicher, einfach und schnell bedienen und einstellen lässt und dabei nur geringe Bedienkräfte erfordert und gleichzeitig sicher und dauerhaft hohe Klemmkräfte erzeugt.

Die Lösung dieser Aufgabe ergibt sich in Verbindung mit den Oberbegriffsmerkmalen erfindungsgemäß aus den Merkmalen des kennzeichnenden Teils des Hauptanspruches, wobei die Schnellspannklammer zur Erzeugung der Spannkraft mit einem Kniehebelverschluss aus zwei über ein Verbindungsgelenk mit-einander verbundene Gelenkhebel ausgestattet ist, die an ihren freien Enden über Anschlussgelenke an mechanisch aus- oder zueinander bewegbaren Bauteilen der Schnellspannklammer angelenkt sind. Mittels eines solchen Kniehebelverschlusses lassen sich alle über Gewinde oder Exzenter betriebenen Klammern von Wundspreizvorrichtungen so umkonstruieren, dass mit nur geringem Kraftaufwand sehr hohe Klemmkräfte verwirklicht werden, sei es für

Klammern für stabförmige runde Haltearme oder kugelförmige Gelenkelemente von solchen Haltearmen.

Ein weiterer Vorteil dieser erfinderischen Schnellspannklammer besteht darin, dass der Betätigungshebel nur über einen kleinen Winkelbereich bewegt werden muss, um aus einer Verstellposition eines geklemmten Halte- oder Verbindungsarmes in eine sehr fest geklemmte Position gebracht zu werden, so dass die Betätigungshebel, wie beispielsweise bei Schraubklemmen der Fall, keinen großen freien Radius zur Betätigung benötigen, der möglicherweise in einem Operationsfeld nicht vorhanden ist.

Vorteilhafte Ausgestaltungen des Gegenstandes der Erfindung ergeben sich mit und in Kombination aus den nachfolgenden Unteransprüchen.

Entsprechend einer besonders bevorzugten Ausführungsform ist der per Hand bedienbare Betätigungshebel unmittelbar an einem der Gelenkhebel des Knieverschlusses, insbesondere einteilig mit diesem ausgeführt, wobei dieser Betätigungshebel in einer Flucht mit dem Verbindungsgelenk und dem diesem näher gelegenen Anschlussgelenk ausgerichtet ist, wodurch sich auch der Bauaufwand der Schnellspannklammer reduziert. In Abhängigkeit von den voraussichtlich zur Verfügung stehenden Platzverhältnisse und Einbausituationen kann ein solcher Betätigungshebel vorteilhafterweise auch seitlich eines Gelenkhebels angeordnet sein, was die Freiheit eines Konstrukteurs wesentlich vergrößert.

Gemäß einer weiteren bevorzugten Ausgestaltung der erfinderischen Schnellspannklammer ist die Aufnahme nach Art einer einen Haltearm oder eine Gelenkkugel teilweise umschließenden Rohrschelle ausgebildet, die Anschlussgelenke an den sich nur geringfügig beabstandet gegenüberliegenden Enden der Rohrschelle aufweist, wobei sich die daran angeordnete Gelenkhebel beide zu einer gemeinsamen Seite in einer annähernd tangentialen Richtung erstrecken und dort über das gemeinsame Verbindungsgelenk miteinander verbunden sind. Die Achsen der Gelenke liegen in einer Klemmposition in einer gemeinsamen Flucht, in der die beiden Achsen der Anschlussgelenke gegeneinandergedrückt gehalten sind.

Um aus dieser Klemmposition in eine nur leicht gelockerte Einstellposition eines Haltearmes in der Schnellspannklammer zu gelangen, muss die Achse des Verbindungsgelenkes in eine nur geringfügig radialere Richtung des Betätigungshebels ausgelenkt werden, was sich mit sehr geringem Kraftaufwand durchführen lässt, ebenso wie sich die erneute feste Klemmposition unter ebenso wenig Kraftaufwand wieder herstellen lässt. Auch körperlich weniger kräftige Operateuren oder Operateurinnen ist damit eine problemlose Benutzung der Schnellspannklammer und damit der gesamten Wundspreizvorrichtung möglich.

Insbesondere zum Austausch von Haltearmen oder zur Reinigung der Wundspreizvorrichtung ist die Rohrschelle vorteilhafterweise in einem den Anschlussgelenken gegenüberliegenden Bereich mit einem weiteren Gelenk oder aber mit zwei mit einem Koppelelement verbundenen Gelenken versehen, so dass bereits ein Öffnungswinkel des Betätigungshebels von etwa 90° dazu führt, dass die Rohrschelle fast vollständig aufgeklappt werden kann, so dass jeder darin gehaltene Haltearm oder jede Gelenkkugel frei daraus entnehmbar ist.

Bei einer besonders bevorzugten Ausführungsform der Erfindung sind drei solcher Schnellspannklammern zu einer Zentralklammer zusammengefasst, wobei zwei parallel angeordnete Schnellspannklammern für zwei Haltearme ausgebildet sind, die dann einen Wundspreizrahmen bilden und die dritte Schnellspannklammer zur Befestigung auf einer Gelenkkugel eines Verbindungsarmes zu einem Zentralhalter eines OP-Tisches ausgebildet ist, so dass bei insgesamt nur geringem Platzbedarf für die Bedienung der Schnellspannklammern ein größtmöglicher Verstellbereich der Haltearme am Verbindungsarm verwirklicht werden kann.

Der erste Gelenkhebel einer Schnellspannklammer besteht dabei aus zwei voneinander beabstandeten Laschen, die beidseitig des ersten Anschlussgelenkes und des Verbindungsgelenkes angeordnet sind, wobei der zweite Gelenkhebel zwischen diesen Laschen angeordnet und stabförmig ausgebildet ist und im gemeinsamen Verbindungsgelenk eine Sperrwelle im zweiten Gelenkhebel angeordnet ist, die eine entsperrbare Rastvorrichtung zur gegenseitigen Bewegungsverriegelung der beiden Gelenkhebel aufweist. Mittels dieser Rastvorrichtung wird es ermöglicht, den Kniehebelverschluss, der in einer Klemmposition quasi selbsthemmend verriegelt, wenn er geringfügig über eine fluchtende Lage der gemeinsamen Gelenkachsen hinaus verschwenkt ist, in einer nur leicht in Öffnungsrichtung verschwenkten Einstellposition eines Haltearmes festzusetzen.

Vorteilhaft ist auch eine andere bandförmige Rohrschelle, die eine Aufnahme ebenfalls umfasst und etwa tangential und über Kreuz verlaufende Fortsätze aufweist, die an ihren Enden die Anschlussgelenke tragen, wobei zwei zueinander abgewinkelte Gelenkhebel so dazwischen und über ein gemeinsames Verbindungsgelenk miteinander verbunden sind, dass bei einer fluchtenden Ausrichtung der drei Gelenke eine Druckkraft in den Gelenkhebeln erzeugt wird, die wiederum Zugkräfte in den Enden der Fortsätze hervorrufen und so die Rohrschelle ähnlich eines zugezogenen Bandes verengen und so eine Klemmkraft erzeugen.

Eine weitere vorteilhafte Variante einer Schnellspannklammer kann in Form einer Spreizzange ausgebildet sein, deren Betätigungsgriffe mit den Anschlussgelenken für die Gelenkhebel versehen sind, die wieder leicht abgewinkelt über ein gemeinsames Verbindungsgelenk miteinander verbunden sind, so dass durch ein in eine gemeinsame Flucht bringen der drei Gelenke eine Klemmkraft auf den als Aufnahme für einen Haltearm oder eine Gelenkkugel eines Haltearmes ausgeführten Zangenkopf erzeugbar ist.

Dieses technische Grundprinzip mittels Kniehebelverschlüssen Schnellspannklammern zu verwirklichen, lässt sich auch für Schnellspannklammern umsetzen, die aus einem U-förmigen Grundkörper bestehen und deren Aufnahmen im Bereich der freien Enden der zueinander parallelen Balken angeordnet sind, wobei sich dahinter ein Spannbolzen quer durch die Balken erstreckt, der sich auf einer Außenseite an einem der Balken abstützt und auf der entgegengesetzten Außenseite ein Anschlussgelenk für einen Gelenkhebel aufweist, der zweite Gelenkhebel an dem diesem Anschlussgelenk zugewandten Balken über ein zweites Anschlussgelenk daran angeordnet ist und beide Gelenkhebel gering-fügig abgewinkelt durch das Verbindungsgelenk miteinander verbunden sind, so dass bei einem in eine gemeinsame Flucht bringen eine die Aufnahme zusammendrückende Klemmkraft erzeugt wird.

Allen denkbaren Ausführungsformen der erfinderischen Schnellspannklammer erlauben bei nur einem geringen Hebelweg eines Betätigungshebels die Erzeugung einer hohen Klemmkraft, wodurch eine hohe Arbeitssicherheit bei der Benutzung der Schnellspannklammern garantiert ist.

Nachfolgend ist ein Ausführungsbeispiel der Erfindung anhand von Zeichnungen näher beschrieben. Es zeigen:

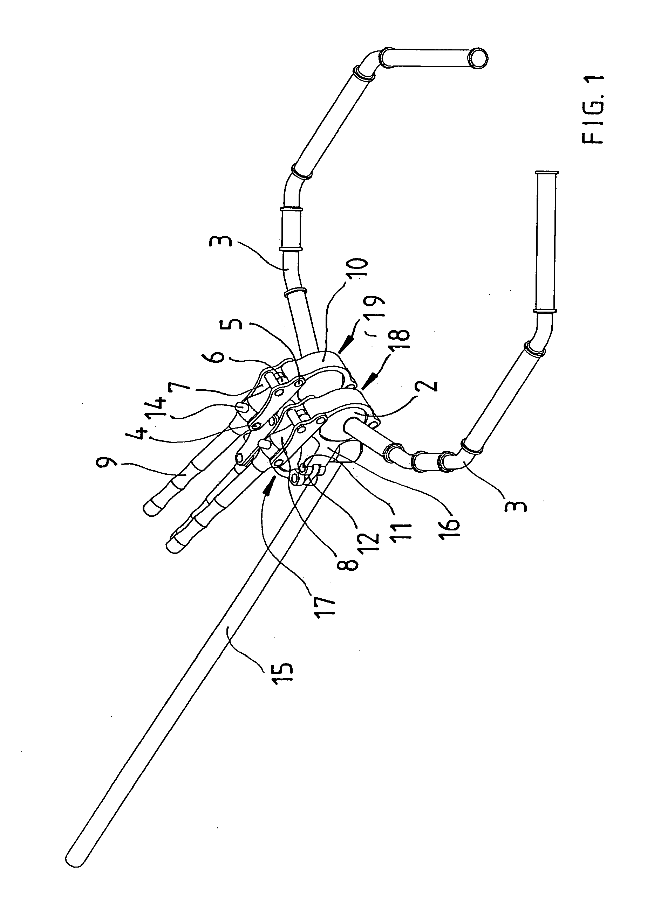

- Fig. 1.: eine Wundspreizvorrichtung mit drei Schnellspannklammern in räumlicher Ansicht,

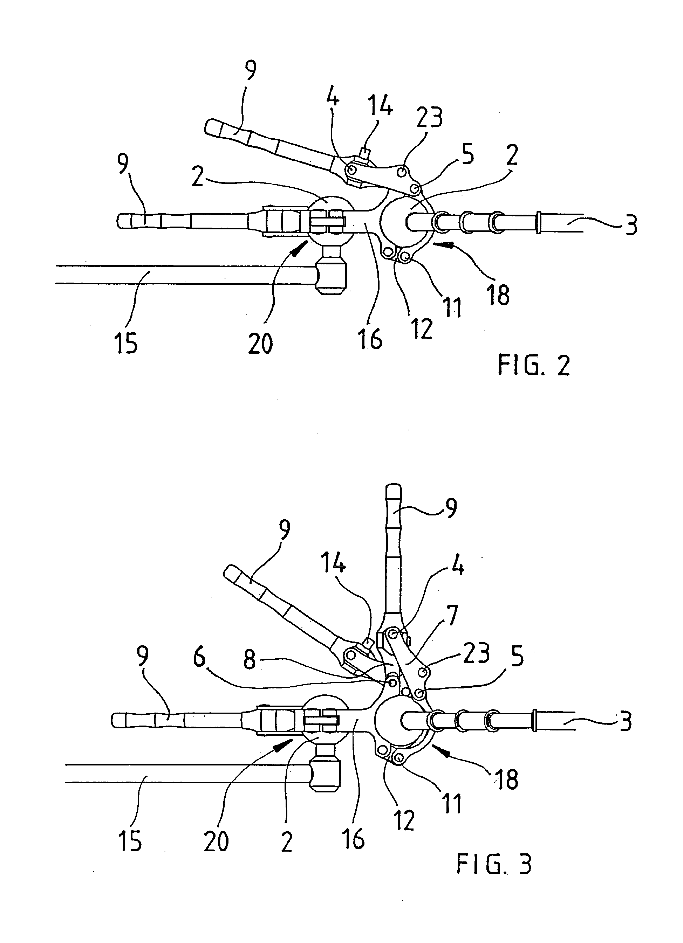

- Fig. 2.: die Wundspreizvorrichtung der Fig. 1 in einer Seitenansicht in Klemmposition aller Schnellspannklammern in Seitenansicht,
- Fig. 3.: die Wundspreizvorrichtung gem. Fig. 2 in einer Einstell- und einer EntnahmePosition der beiden Schnellspannklammern der Haltearme und

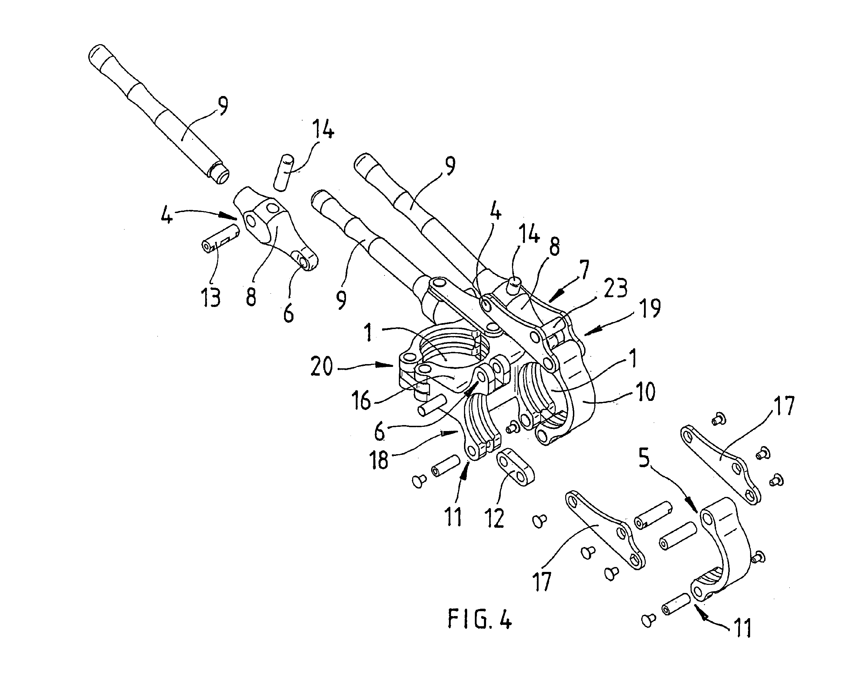

- Fig. 4.: eine Explosionszeichnung einer Schnellspannklammer einer aus drei Schnellspannklammern bestehenden Zentralklammer in 3-D-Ansicht.

Die dargestellte Wundspreizvorrichtung besteht aus einer Zentralklammer 16 mit drei Schnellspannklammern 18;19;20, die jeweils eine Gelenkkugel 2 klemmen, von denen diejenige der der hinteren Schnellspannklammer 20 zugeordneten Gelenkkugel 2 mit einem Verbindungsarm 15 zur Befestigung der Wundspreizvorrichtung an einem Zentralhalter an einem OP-Tisch zugehörig ist und die beiden anderen Gelenkkugeln 2 Haltearmen 3, die einen Wundspreizrahmen aufspannen.

Jede einzelne der drei vorhandenen Schnellspannklammern 18;19;29 ist nach Art einer Rohrschelle 10 ausgebildet, die jeweils als Aufnahme 1 für eine Gelenkkugel 2 ausgebildet ist und diese fast vollständig umschließt, wobei die Anschlussgelenke 5;6 an den sich nur geringfügig beabstandet gegenüberliegenden Enden der Rohrschelle 10 angeordnet sind, in denen die Gelenkhebel 7;8 sich beide gemeinsam nach hinten in eine etwa tangentiale Richtung erstrecken und dort über das Verbindungsgelenk 4 miteinander verbunden sind. Der Gelenkhebel 7 besteht dabei aus zwei separaten Laschen 17, die beidseitig außen an dem ersten Anschlussgelenk 5 sowie dem als Stab ausgebildeten zweiten Gelenkhebel 8 festgelegt sind, der sich gradlinig weiter nach hinten erstreckt und den Betätigungshebel 9 bildet.

Die beiden den Gelenkhebel 7 erzeugenden Laschen 17 sind mit einer weiteren Halteachse 23 verbunden, die gleichzeitig als Bewegungsbegrenzung des Betätigungshebels 9 in Schließrichtung dient und zwar für geringfügig über eine Klemmposition hinaus, so dass in der Klemmposition des Betätigungshebels 9 eine Art Selbsthemmung des Kniehebelverschlusses erzeugt ist.

Die Achse des Verbindungsgelenkes 4 ist in den Laschen 17 verdrehfest gelagert und als Sperrwelle 13 so ausgeführt, dass sich ein Verschwenken des Betätigungshebels 9 und damit des Gelenkhebels 8 um das Verbindungsgelenk 4 herum durch einen Sperrbolzen 14 verriegeln lässt, der korrespondierende Rastflächen mit der Sperrwelle 13 aufweist. Der Betätigungshebel 9 ist in einer Einstell-Position eines Haltearmes geklemmt, so dass sich ohne Betätigung des Sperrbolzens 14 der Kniehebelverschluss nur begrenzt öffnen lässt, d.h. sich die Haltearme gegen eine leichte Reibungskraft bewegen und einstellen, aber nicht aus der Aufnahme 1 entfernen lassen. Nach Betätigung des Sperrbolzens 14 kann der Kniehebelverschluss komplett geöffnet und die Gelenkkugel 2 aus der Aufnahme 1 entfernt werden.

Zum leichteren Austausch der Gelenkkugel 2 ist die Rohrschelle in einem den Anschlussgelenken 5;6 entgegengesetzten Bereich mit zwei weiteren Gelenken 11 und einem dazwischen angeordneten Koppelelement 12 versehen, wodurch insbesondere bei der in den Fig. dargestellten Zentralklammer 16 ein quasi fester Klammerteil und drei daran angelenkte Spannbügel als bewegliche Bauteile vorhanden sind.

Zeichnerisch nicht dargestellt sind weitere Schnellspannklammern, bei denen über Gewinde oder Exzenter funktionierende Klemmverfahren durch Kniehebelverschlüsse ersetzt sind, was bei allen bekannten Schnellspannklammern erfolgen kann.

## Patentansprüche

1. Schnellspannklammer einer Wundspreizvorrichtung mit mindestens einer verspannbaren Aufnahme für mindestens einen Haltearm oder eine Gelenkkugel eines Haltearmes, wobei die Schnellspannklammer zur Erzeugung der Spannkraft mit einem Kniehebelverschluss aus zwei mittels eines Verbindungsgelenks (4) miteinander verbundenen Gelenkhebeln (7;8) ausgestattet ist, die an ihren freien Enden über Anschlussgelenke (5;6) an mechanisch aus- oder zueinander bewegbaren Bauteilen der Schnellspannklammer (17;18;19) angelenkt sind und ein Gelenkhebel (8) einen per Hand bedienbaren Betätigungshebel (9) aufweist, wobei der erste Gelenkhebel (7) aus zwei voneinander beabstandeten Laschen (17) besteht, die beidseitig des ersten Anschlussgelenks (5) und des Verbindungsgelenks (4) angeordnet sind und im Verbindungsgelenk (4) eine Sperrwelle (13) angeordnet ist, die eine entsperrbare Rastvorrichtung zur gegenseitigen Bewegungsverriegelung der beiden Gelenkhebel (7;8) aufweist, **dadurch gekennzeichnet, dass** die Sperrwelle (13) verdrehfest in den Laschen (17) des ersten Gelenkhebels (7) gelagert ist und sich rechtwinklig und achsversetzt ein lösbarer Sperrbolzen (14) quer zur Sperrwelle (13) durch das Verbindungsgelenk (4) erstreckt, der im unbetätigten Zustand eine begrenzte Öffnung der Schnellspannklammer erlaubt, und damit die Schnellspannklammer (17;18;19) unter nur so leichter Klemmkraft verriegelt, dass die Haltearme gegen eine leichte Reibungskraft beweg- und einstellbar, aber aus der Aufnahme 1 unentfernbar sind.

2. Schnellspannklammer nach Anspruch 1, **dadurch gekennzeichnet, dass** der Betätigungshebel (9) in einer Flucht mit dem Verbindungsgelenk (4) und dem näher gelegenen Anschlussgelenk (6) ausgerichtet ist.

3. Schnellspannklammer nach einem der vorgenannten Ansprüche, **dadurch gekennzeichnet, dass** die Aufnahme (1) nach Art einer einen Haltearm (3) oder eine Gelenkkugel (2) teilweise umschließenden Rohrschelle (10) ausgebildet ist, die Anschlussgelenke (5;6) an den sich nur geringfügig beabstandet gegenüberliegenden Ende der Rohrschelle (10) angeordnet sind und sich beide Gelenkhebel (7;8) zu einer gemeinsamen Seite erstrecken und dort über das Verbindungsgelenk (4) miteinander verbunden sind, wobei die Achsen der Gelenke (4;5;6) in einer Klemmposition in einer Flucht liegen und die Achsen der beiden Anschlussgelenke (5;6) aufeinander zu gedrückt gehalten sind und in einer Einstell- oder Entnahme- Position eines Haltearmes (3) die Achse des Verbindungsgelenkes (4) geringfügig oder stärker aus dieser Lage in eine radialere Richtung ausgelenkt ist.

4. Schnellspannklammer nach Anspruch 3, **dadurch gekennzeichnet, dass** die Rohrschelle (10) in einem den Anschlussgelenken (5;6) entgegengesetzten Bereich mit einem weiteren Gelenk (11) oder mit zwei mit einem Koppelelement (12) verbundenen Gelenken (11) versehen ist.

5. Schnellspannklammer nach Anspruch 1, **dadurch gekennzeichnet, dass** sie als Zentralklammer mit drei Schnellspannklammern (17;18;19) für zwei Haltearme (3) und einen Verbindungsarm (15) zu einem Zentralhalter eines OP-Tisches ausgebildet ist.

6. Schnellspannklammer nach Anspruch 1, **dadurch gekennzeichnet, dass** eine bandförmige Rohrschelle eine Aufnahme (1) etwa vollständig umfasst und etwa tangential und über Kreuz verlaufende Fortsätze aufweist, die die Anschlussgelenke (5;6) tragen und dass zwei abgewinkelte Gelenkhebel (7;8) so dazwischen über ein gemeinsames Verbindungsgelenk (4) miteinander verbunden sind, dass bei einer Ausrichtung der drei Gelenke (4;5;6) in eine Flucht Druckkräfte in den Gelenkhebeln (7;8) erzeugt sind, die Zugkräfte in den Enden der Fortsätze hervorrufen, die die Rohrschelle verengen.

7. Schnellspannklammer nach Anspruch 1, **dadurch gekennzeichnet, dass** sie in Form einer Spreizzange ausgebildet ist, deren Betätigungsgriffe mit den Anschlussgelenken versehen sind, so dass durch das in eine Flucht bringen von den Anschlussgelenken und dem Verbindungsgelenk eine Klemmkraft auf den als Aufnahme ausgeführten Zangenkopf erzeugt ist.

8. Schnellspannklammer nach Anspruch 1, **dadurch gekennzeichnet, dass** sie einen U-förmigen Grundkörper aufweist und die Aufnahme (1) im Bereich der Enden der zueinander parallelen Balken angeordnet ist und sich dahinter ein Spannbolzen quer durch die Balken erstreckt, der sich auf einer Außenseite an einem der Balken abstützt und auf der entgegengesetzten Außenseite ein Anschlussgelenk für einen Gelenkhebel aufweist, der zweite Gelenkhebel an dem diesen Anschlussgelenk zugewandten Balken an einem zweiten Anschlussgelenk angeordnet ist und beide Gelenkhebel abgewinkelt durch das Verbindungsgelenk miteinander verbunden sind und dass durch ein in eine gemeinsame Flucht bringen der Gelenke eine die Aufnahme (1) zusammendrückende Klemmkraft erzeugt ist.

## Claims

1. Quick-action clamping clip of a wound retractor, comprising at least one clampable receptacle for at least one holding arm or a joint ball of a holding arm, wherein the quick-action clamping clip, for producing the clamping force, is provided with a toggle fastener consisting of two articulated levers (7; 8) which are connected to one another by means of a connecting joint (4) and which are linked at their free ends via attachment joints (5; 6) to components of the quick-action clamping clip (17; 18; 19) which can be mechanically moved away from or toward one another and one articulated lever (8) has an actuating lever (9) which can be operated manually, wherein the first articulated lever (7) consists of two spaced-apart straps (17) which are arranged on both sides of the first attachment joint (5) and of the connecting joint (4), and a locking shaft (13) is arranged in the connecting joint (4), said locking shaft (13) having a releasable latching device for mutually locking the movement of the two articulated levers (7; 8), **characterized in that** the locking shaft (13) is mounted in the straps (17) of the first articulated lever (7) in a rotationally fixed manner, and a releasable locking pin (14) extends through the connecting joint (4) at right angles and in an axially offset manner transversely to the locking shaft (13), which locking pin (14), in the non-actuated state, permits limited opening of the quick-action clamping clip and thus locks the quick-action clamping clip (17; 18; 19) with only such slight clamping force that the holding arms can be moved and set against a slight frictional force but cannot be removed from the receptacle (1).

2. Quick-action clamping clip according to Claim 1, **characterized in that** the actuating lever (9) is oriented in alignment with the connecting joint (4) and the attachment joint (6) closer to said connecting joint (4).

3. Quick-action clamping clip according to one of the aforesaid claims, **characterized in that** the receptacle (1) is designed like a pipe clamp (10) partly enclosing a holding arm (3) or a joint ball (2), the attachment joints (5; 6) are arranged on the ends of the pipe clamp (10) located opposite one another at only a slight distance apart, and both articulated levers (7; 8) extend to a common side and are connected to one another there via the connecting joint (4), wherein the pivots of the joints (4; 5; 6) are in alignment in a clamping position, and the pivots of the two attachment joints (5; 6) are held so as to be pressed toward one another, and, in a setting or removal position of a holding arm (3), the pivot of the connecting joint (4) is deflected slightly or to a greater extent from this position further in a radial direction.

4. Quick-action clamping clip according to Claim 3, **characterized in that** the pipe clamp (10), in a region opposite the attachment joints (5; 6), is provided with a further joint (11) or with two joints (11) connected by a coupling element (12).

5. Quick-action clamping clip according to Claim 1, **characterized in that** it is designed as a central clip having three quick-action clamping clips (17; 18; 19) for two holding arms (3) and a connecting arm (15) to a central holder of an operating table.

6. Quick-action clamping clip according to Claim 1, **characterized in that** a band-shaped pipe clamp encloses a receptacle (1) approximately completely and has extensions which run approximately tangentially and crosswise and which carry the attachment joints (5; 6), and **in that** two angled articulated levers (7; 8) are connected to one another in between via a common connecting joint (4) in such a way that compressive forces are produced in the articulated levers (7; 8) when the three joints (4; 5; 6) are in alignment and these compressive forces cause tensile forces in the ends of the extensions, said tensile forces constricting the pipe clamp.

7. Quick-action clamping clip according to Claim 1, **characterized in that** it is designed in the form of spreading forceps, the actuating handles of which are provided with the attachment joints, such that, by the attachment joints and the connecting joint being brought into alignment, a clamping force is produced on the head of the forceps, which is designed as a receptacle.

8. Quick-action clamping clip according to Claim 1, **characterized in that** it has a U-shaped basic body and the receptacle (1) is arranged in the region of the ends of the beams which are parallel to one another, a clamping pin extends therebehind transversely through the beams, said clamping pin being supported on an outer side on one of the beams and having on the opposite outer side an attachment joint for an articulated lever, the second articulated lever is arranged on the beam facing this attachment joint at a second attachment joint, and both articulated levers are connected to one another in an angled manner by the connecting joint, and **in that** the joints, by being brought into alignment, produce a clamping force which compresses the receptacle (1).

## Revendications

1. Pince de serrage rapide d'un écarteur de plaie comprenant au moins un logement, pouvant être serré, pour au moins un bras de retenue ou une rotule d'articulation d'un bras de retenue, la pince de serrage rapide, pour générer la force de serrage, étant pourvue d'une fermeture à genouillère constituée de deux leviers articulés (7 ; 8) connectés l'un à l'autre au moyen d'une articulation de liaison (4) qui sont articulés au niveau de leurs extrémités libres par le biais d'articulations de raccordement (5 ; 6) à des composants de la pince de serrage rapide (17 ; 18 ; 19) déplaçables mécaniquement l'un de l'autre ou l'un vers l'autre et un levier d'articulation (8) présentant un levier d'actionnement (9) pouvant être actionné à la main, le premier levier d'articulation (7) se composant de deux pattes (17) espacées l'une de l'autre qui sont disposées de chaque côté de la première articulation de raccordement (5) et de l'articulation de liaison (4) et un arbre de verrouillage (13) étant disposé dans l'articulation de liaison (4), lequel présente un dispositif d'encliquetage déverrouillable pour le verrouillage mutuel du mouvement des deux leviers d'articulation (7 ; 8), **caractérisée en ce que** l'arbre de verrouillage (13) est supporté de manière fixée en rotation dans les pattes (17) du premier levier d'articulation (7) et un boulon de verrouillage amovible (14) s'étend à angle droit et avec son axe décalé transversalement à l'arbre de verrouillage (13) à travers l'articulation de liaison (4), lequel boulon permet, dans l'état non actionné, une ouverture limitée de la pince de serrage rapide et verrouille ainsi la pince de serrage rapide (17 ; 18 ; 19) avec une force de serrage seulement assez faible pour que les bras de retenue puissent être déplacés et ajustés à l'encontre d'une légère force de friction mais ne puissent pas être enlevés du logement (1).

2. Pince de serrage rapide selon la revendication 1, **caractérisée en ce que** le levier d'actionnement (9) est orienté en affleurement avec l'articulation de liaison (4) et l'articulation de raccordement (6) plus proche.

3. Pince de serrage rapide selon l'une quelconque des revendications précédentes, **caractérisée en ce que** le logement (1) est réalisé à la manière d'un collier de serrage (10) entourant partiellement un bras de retenue (3) ou une rotule d'articulation (2), les articulations de raccordement (5 ; 6) sont disposées au niveau des extrémités opposées seulement légèrement espacées du collier de serrage (10) et les deux leviers d'articulation (7 ; 8) s'étendent jusqu'à un côté commun et sont connectés l'un à l'autre à cet endroit par le biais de l'articulation de liaison (4), les axes des articulations (4 ; 5 ; 6) étant situés en affleurement dans une position de serrage et les axes des deux articulations de raccordement (5 ; 6) étant maintenus pressés l'un vers l'autre et, dans une position d'ajustement ou d'enlèvement d'un bras de retenue (3), l'axe de l'articulation de liaison (4) étant légèrement dévié ou étant plus fortement dévié hors de cette position dans une direction plus radiale.

4. Pince de serrage rapide selon la revendication 3, **caractérisée en ce que** le collier de serrage (10), dans une région opposée aux articulations de raccordement (5 ; 6), est pourvu d'une articulation supplémentaire (11) ou de deux articulations (11) connectées par un élément d'accouplement (12).

5. Pince de serrage rapide selon la revendication 1, **caractérisée en ce qu'**elle est réalisée sous forme de pince centrale avec trois pinces de serrage rapide (17 ; 18 ; 19) pour deux bras de retenue (3) et un bras de liaison (15) à un dispositif de retenue central d'une table d'opération.

6. Pince de serrage rapide selon la revendication 1, **caractérisée en ce qu'**un collier de serrage en forme de bande entoure approximativement complètement un logement (1) et présente des saillies s'étendant approximativement tangentiellement et de manière croisée, qui portent les articulations de raccordement (5 ; 6), et **en ce que** deux leviers d'articulation coudés (7 ; 8) sont connectés l'un à l'autre entre celles-ci par le biais d'une articulation de liaison commune (4) de telle sorte que dans le cas d'une orientation des trois articulations (4 ; 5 ; 6) en affleurement, des forces de pression soient générées dans les leviers d'articulation (7 ; 8), lesquelles provoquent des forces de traction dans les extrémités des saillies, qui resserrent le collier de serrage.

7. Pince de serrage rapide selon la revendication 1, **caractérisée en ce qu'**elle est réalisée sous la forme d'une pince d'écartement dont les prises d'actionnement sont pourvues des articulations de raccordement de telle sorte que par le fait d'amener en affleurement les articulations de raccordement et l'articulation de liaison, une force de serrage soit générée sur la tête de pince réalisée sous forme de logement.

8. Pince de serrage rapide selon la revendication 1, **caractérisée en ce qu'**elle présente un corps de base en forme de U et le logement (1) est disposé dans la région des extrémités des poutres mutuellement parallèles et un boulon de serrage s'étend derrière lui transversalement à travers les poutres, lequel s'appuie sur un côté extérieur contre l'une des poutres et présente, sur le côté extérieur opposé, une articulation de raccordement pour un levier d'articulation, le deuxième levier d'articulation est disposé au niveau de la poutre tournée vers cette articulation de raccordement contre une deuxième articulation de raccordement et les deux leviers d'articulation sont connectés l'un à l'autre, de manière coudée, par l'articulation de liaison et **en ce qu'**une force de serrage comprimant le logement (1) est générée par le fait d'amener les articulations en affleurement l'une avec l'autre.
